(19) Europäisches Patentamt
European Patent Office
Office européen des brevets

(11) EP 2 496 552 B1

(12) EUROPEAN PATENT SPECIFICATION

(45) Date of publication and mention of the grant of the patent:
**13.07.2016 Bulletin 2016/28**

(21) Application number: **10774384.1**

(22) Date of filing: **27.10.2010**

(51) Int Cl.:
*C07C 217/42* (2006.01) *C07C 229/16* (2006.01)
*C07C 239/12* (2006.01) *A01N 33/12* (2006.01)
*A61Q 5/02* (2006.01) *A61Q 5/12* (2006.01)
*C08L 63/00* (2006.01) *A01N 25/30* (2006.01)
*A01N 57/20* (2006.01) *A61K 8/41* (2006.01)
*C11D 1/42* (2006.01) *C11D 1/62* (2006.01)
*C11D 1/75* (2006.01) *D06M 13/332* (2006.01)
*D06M 13/467* (2006.01)

(86) International application number:
**PCT/US2010/054196**

(87) International publication number:
**WO 2011/056620 (12.05.2011 Gazette 2011/19)**

(54) **DIFUNCTIONAL, AMINE-BASED SURFACTANTS, AND THEIR PRECURSORS, PREPARATION, COMPOSITIONS AND USE**

BIFUNKTIONELLE TENSIDE AUF AMINBASIS UND IHRE VORLÄUFER, ZUBEREITUNG, ZUSAMMENSETZUNGEN UND VERWENDUNG

TENSIO-ACTIFS DIFONCTIONNELS À BASE D'AMINE ET LEURS PRÉCURSEURS, LEUR PRÉPARATION, LEURS COMPOSITIONS ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.11.2009 US 258803 P**

(43) Date of publication of application:
**12.09.2012 Bulletin 2012/37**

(73) Proprietor: **Dow Global Technologies LLC**
**Midland, MI 48674 (US)**

(72) Inventors:
- **DROVETSKAYA, Tatiana**
  **Basking Ridge**
  **NJ 07920 (US)**
- **WASSERMAN, Eric, P.**
  **Hopewell**
  **NJ 08525 (US)**
- **FRANK, Diane**
  **Woodbridge**
  **NJ 07095 (US)**
- **JORDAN, Susan, L.**
  **Doylestown**
  **PA 18902 (US)**
- **WANGLIN, Yu**
  **Midland**
  **MI 48642 (US)**
- **RAND, Cynthia**
  **Sanford**
  **MI 48657 (US)**
- **GRAF, Irina, V.**
  **Lake Jackson**
  **TX 77566 (US)**
- **DAUGS, Edward, D.**
  **Midland**
  **MI 48642 (US)**

(74) Representative: **Beck Greener**
**Fulwood House**
**12 Fulwood Place**
**London WC1V 6HR (GB)**

(56) References cited:
**US-A- 3 959 461 US-A- 5 705 681**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MORREN H ET AL: "ANTISPASMODIQUES A ACTION MUSCULOTROPE DERIVES DU 1,3-BIS (DIALCOYLAMINO)-PROPANE", INDUSTRIE CHIMIQUE BELGE - BELGISCHE CHEMISCHE INDUSTRIE, FEDERATION DES INDUSTRIES CHIMIQUES DE BELGIQUE, BRUXELLES, BE, vol. 20, no. 7, 1 January 1955 (1955-01-01), pages 733-745, XP009147067, ISSN: 0019-9052
- BAKKER P M ET AL: "AN EXPLORATORY STUDY OF THE ADDITION REACTIONS OF ETHYLENEGLYCOL, 2-CHLOROETHANOL AND 1,3-DICHLORO-2-PROPANOL TO 1-DODECENE", JOURNAL OF THE AMERICAN OIL CHEMISTS' SOCIETY, SPRINGER, BERLIN, DE, vol. 44, no. 9, 1 January 1967 (1967-01-01), pages 517-518, XP009081570, ISSN: 0003-021X, DOI: DOI:10.1007/BF02679238

**Description**

FIELD OF THE INVENTION

The invention relates to a new class of amine containing bicephalic surfactants that are characterized by being double-headed, single-chain surfactants. The bicephalic amine derivatives are novel structures that can be converted to useful surfactant derivatives, e.g., bis-quaternary ammonium salts, amine oxides, or inner salts, and the like. In one aspect the invention relates to the compositions of matter for the diamine precursors of these surfactants while in another aspect, the invention relates to the process for preparing these surfactants. In still another aspect the invention relates to compositions comprising these surfactants while in yet another aspect, the invention relates to various applications in which these surfactants are used.

BACKGROUND OF THE INVENTION

Amine-based surfactants have found widespread use in a number of applications including, but not necessarily limited to, disinfectants, cleansers and sterilizers, cosmetics (deodorants, dandruff removers, emulsion stabilizers), fungicides, mildew preventatives, antistatic and sizing additives, biocides, to increase the affinity of dyes for photographic film, to improve dispersability in the coatings of pigment particles, to increase adhesion of road dressings and paints, as adjuvants in agricultural applications, and in applications related to oil and gas production and transportation including, but not necessarily limited to, surfactants, dispersants, biocides, corrosion inhibitors, *etc.* For example, US 3,959,461 discloses quaternary ammonium salts prepared from an amino amide and an amino ester that are found to be useful in hair cream rinse formulations. Most of the known amine-based surfactants comprise a polymeric backbone with one functional group per molecule (monofunctional) or three or more groups per molecule (polyfunctional). In contrast, the amine-based surfactants of this invention comprise two functional groups (difunctional), and this difunctionality imparts unique properties to these compounds. US Patent 5,705,681 discloses diquaternary ammonium esters which are useful as bleach activators.

Conditioning is one of the key recognized consumer benefits for hair care products. It is typically achieved through deposition of conditioning agents on hair from leave-on and rinse-off treatments. While depositing conditioning materials from a leave-on treatment is more straightforward, deposition in a rinse-off format presents a challenge. When a rinse-off treatment, such as, for example, shampoo or conditioner, is applied, only a small portion of the conditioning benefit agents contained in the formulation adsorb to the hair surface and stay behind after the rinse-off cycle is complete. Some cationic materials, e.g., the mono-quats (mono-quaternary ammonium surfactants) and poly-quats (polyquaternary ammonium surfactants), can be used for these purposes as they deposit and stay on hair more easily due to the anionic charge of the hair surface. Nevertheless, an interest remains in the personal care industry to develop cationic materials that are even more efficient than the ones that already exist, and for materials that will perform universally well in leave-on and rinse-off shampoo and conditioner type of systems.

Herbicide compositions often include components (commonly termed adjuvants) that enhance the performance and absorption into the plant of the active ingredient. Surfactants are frequently used as adjuvants because they can both enhance the absorption of the active ingredient into the plant as well as facilitate the application of the herbicide. Surfactant adjuvants are often amine-based.

Other applications in which amine-based surfactants have proven useful include the catalysis of addition polymerizations, e.g., epoxy polymerizations, and cold water cleaning, especially the removal of nitrogenous oxides.

SUMMARY OF THE INVENTION

In one embodiment the invention is a new class of difunctional amines, and their derivatives: difunctional cationic surfactants, such as di-quats (di- or bis-quaternary ammonium surfactants), difunctional amphoteric surfactants, such as di-betaines, and difunctional nonionic surfactants, such as bis-N-oxides, having bicephalic structures.

In one embodiment the invention is the diamine precursors of this new class of amine-based surfactants. These precursors are compounds of formula I.

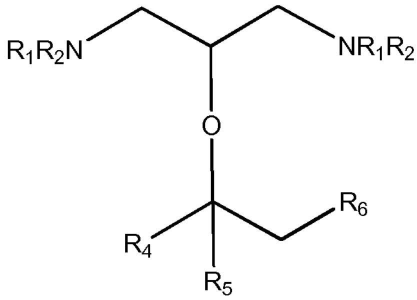

(I)

in which $R_1$, $R_2$, $R_4$, $R_5$ and $R_6$ are as defined below.

In one embodiment the invention is the cationic surfactant of formula II (below). In one embodiment the invention is a process of preparing the cationic surfactant of formula II from the amine precursor of formula I, the process comprising the step of contacting under ionizing conditions (i) a diamine of formula I, and (ii) a haloalkyl compound. The process is generally known as amine alkylation.

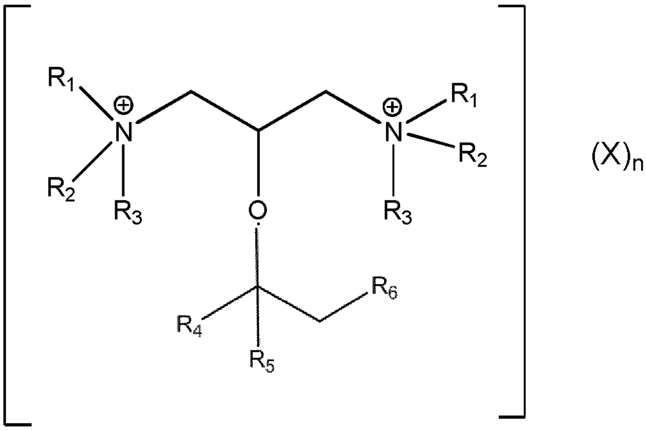

(II)

in which $R_1$, $R_2$, $R_3$, $R_4$, $R_5$, $R_6$, X and n are as defined below.

In one embodiment the invention is the amphoteric surfactant of formula III (below). In one embodiment the invention is a process of preparing the amphoteric surfactant of formula III (below) from the amine precursor of formula I, the process comprising the step of contacting the amine precursor of formula I with an alkylating agent.

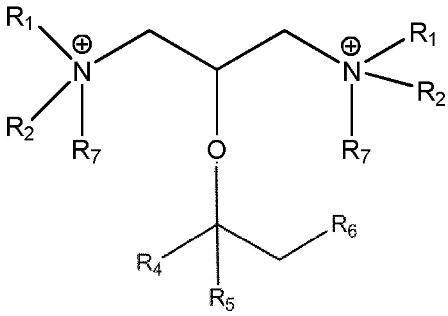

(III)

in which $R_1$, $R_2$, $R_4$, $R_5$, $R_6$ and $R_7$ are as defined below.

In one embodiment the invention is a nonionic surfactant of formula IV (below). In one embodiment the invention is a process of preparing the nonionic surfactant of formula IV from the amine precursor of formula I, the process comprising the step of contacting the amine precursor with an oxidizing agent.

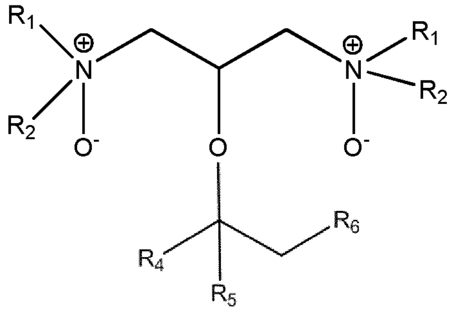

(IV)

in which $R_1$, $R_2$, $R_4$, $R_5$, $R_6$ are as defined below.

In one embodiment the invention is a composition comprising one or more amine based surfactants of formula II, III, or IV.

In one embodiment the invention is the use of the amine-based surfactant, alone or in a composition, in any one of a number of different applications including but not limited to personal care (specifically including hair cleansing), cleaning applications (specifically hard surface and laundry), agricultural treatments (specifically herbicide formulations), and addition polymerization catalysis (specifically including epoxy polymerizations).

DESCRIPTION OF THE PREFERRED EMBODIMENT

*Definitions*

Unless stated to the contrary, implicit from the context, or customary in the art, all parts and percents are based on weight and all test methods are current as of the filing date of this disclosure.

*Cationic Surfactants*

In one embodiment the invention is a new class of cationic surfactants. The cationic surfactants of the invention contain two quaternary ammonium groups on a lightly branched alkyl ether backbone. By "lightly branched" is meant that the alkyl ether backbone contains alkyl groups as defined by $R_4$, $R_5$ and $R_6$. The cationic surfactants exhibit an improved spectrum of properties relative to mono-quat and poly-quat surfactants allowing them to be used in a wide variety of applications. For instance, surfactants of the invention exhibit an improved spectrum of properties relative to mono-quats in conditioners and shampoos, and an improved spectrum of properties relative to poly-quats in conditioners and comparable performance to poly-quats in shampoo.

The cationic surfactants of the invention are compounds of formula II:

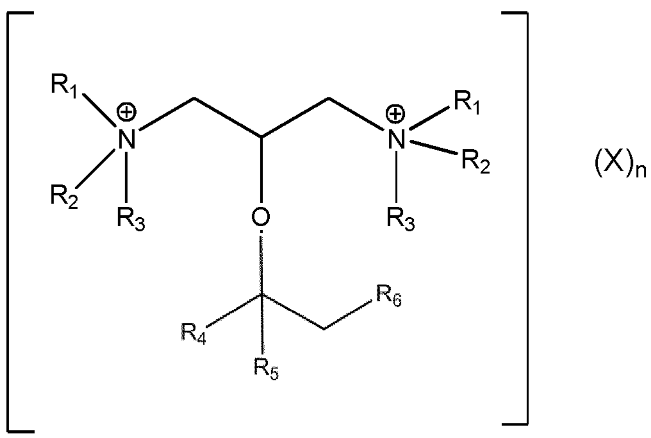

(II)

in which $R_1$ and $R_2$ are $C_1$-$C_4$ linear or branched alkyl groups, or may be joined with one another to form a ring of 3-9 carbon atoms;

$R_3$ is a $C_1$-$C_{20}$ linear or branched alkyl or arylalkyl group;

$R_4$ is hydrogen or a $C_1$-$C_8$ alkyl group, preferably hydrogen;
$R_5$ is a $C_1$-$C_{10}$ linear alkyl group;
$R_6$ is a linear $C_2$-$C_{20}$ alkyl group, and $R_5$ and $R_6$ may be joined with one another to form a ring of 3-10 carbon atoms; with the proviso that the combined carbon atom count of $R_4$, $R_5$, $R_6$ and the central carbon atom is 4 to 22;
X is a mono-or di-anion of at least one of halide (chloride, bromide, or iodide), bicarbonate, carbonate, sulfate, methyl sulfate and bisulfate; and
n is an integer such that the total anionic charge provided by $X_n$ = 2.

In one embodiment $R_5$ and $R_6$ can join to form a ring of 3-10 carbon atoms.

The processes described below for preparing the cationic surfactants of this invention may result in the formation of mixtures of compounds of formula I. Although the individual compounds of formula I may be isolated from the mixture, this step is not necessary, and indeed sometimes the surfactant is preferably used in the form of the mixture. Accordingly, surfactants that are mixtures of compounds of formula I are contemplated and are within the scope of the invention.

Preferred cationic surfactants of formula I include N,N,N,N',N',N'-hexamethyl-2-(1-methylundecyloxy)-1,3-propanediaminium dichloride; N,N,N,N',N',N'-hexamethyl-2-(1-methylpentadecyloxy)-1,3-propanediaminium dichloride; N,N,N,N',N',N'-hexamethyl-2-(1-methyltridecyloxy)-1,3-propanediaminium dichloride; N,N,N,N',N',N'-hexamethyl-2-(1-methylheptadecyloxy)-1,3-propanediaminium dichloride; N,N,N,N',N',N'-hexaethyl-2-(1-methylpentadecyloxy)-1,3-propanediaminium dichloride; N,N,N',N'-tetraethyl-N,N'-dimethyl-2-(1-methylpenta-decyloxy)-1,3-propanediaminium dichloride; and N,N,N',N'-tetraethyl-N,N'-dibenzyl-2-(1-methylpentadecyloxy)-1,3-propanediaminium dichloride and their similar structural isomers in which the alkoxy group is substituted on any secondary carbon of the alkyl chain. Other preferred cationic surfactants of formula II include N,N,N,N',N',N'-hexamethyl-2-(1-ethyldecyloxy)-1,3-propanediaminium dichloride, and N,N,N,N',N',N'-hexamethyl-2-(1-ethyltetradecyloxy)-1,3-propanediaminium dichloride, and similar structural isomers in which the alkoxy group is substituted on any secondary carbons of the alkyl chain.

In one embodiment the invention is a process for making the cationic surfactants of formula II, the process comprising the step of contacting under ionizing conditions (i) a diamine of formula I, and (ii) a haloalkyl compound. "Ionizing conditions" include a temperature of 20 to 250, preferably 35 to 200 and more preferably 50 to 150, °C. Ionizing conditions may also include a solvent to facilitate the reaction, e.g., water, alcohols, ethers, nitriles, N,N-dialkylamides and the like, or mixtures thereof. The upper limit on the temperature is such as to avoid Hoffman degradation of the reaction product. Pressure is a function of the solvent and alkylating agent. The optional solvent should be sufficiently polar to allow the intermediate mono-quat to remain in solution at the reaction temperature. Residence time is a function of the substrates and temperature. Typically, the reaction time is sufficient to completely convert the diamine to di-quat, and this time is generally greater than one hour and can be as long as 24 hours or more. The process is usually performed without a catalyst.

The ether component of the diamine precursor of formula I may be prepared as described in US-A-2009/0281359, filed April 27, 2009. Generally, the synthesis comprises the reaction of an alcohol compound with an olefin in the presence of an acidic etherification catalyst. Typically, an equimolar or slight excess of the olefin is used. A solvent may be used, although not required. The reaction may be conducted at elevated temperature, such as 50 to 150 °C. Once the desired amount of the ether compound product is formed (as determined, for instance, by gas chromatography), the reaction mixture is cooled and subjected to conventional workup. For instance, for removal of a homogeneous acid catalyst, the cooled mixture is added to water containing bicarbonate and/or chloride salts, and the organic liquid layer of the mixture containing the ether compound removed. The ether compound may be further purified by known techniques, such as distillation.

The alcohol of the above-described synthesis is generally of formula (V):

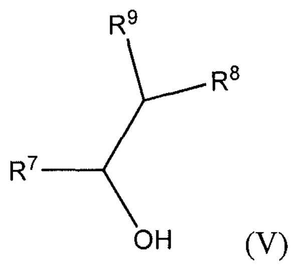

(V)

in which $R^7$ is H or $CH_2X$, $R^8$ is X, and $R^9$ is H or $CH_2X$, wherein one of $R^7$ or $R^9$ is H and X is F, Cl, Br, or I (preferably Cl).

Preferred alcohols for the synthesis include: 1,3-dihalo-2-propanol and 2,3-dihalopropanol, or a mixture of the two. Particularly preferred are 1,3-dichloro-2-propanol and 2,3-dichloropropanol, or a mixture of the two.

The olefin for use in the above synthesis is preferably a linear or branched alphaolefin (i.e., 1-alkene) containing

4 to 22 carbon atoms, or a mixture of isomers of linear or branched 1-alkenes containing 4 to 22 carbon atoms together with their internal and/or tertiary olefin isomers. Preferably, the alkenes are linear and contain 6 to 18 carbon atoms. Non-limiting examples of particularly preferred alpha olefins include: 1-butene, 1-pentene, 1-hexene, 4-methyl-1-pentene, 1-heptene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, 1-eicosene, or mixtures of two or more of these compounds.

As the olefin may be isomerized when contacted with the acidic etherification catalyst, the use of an alpha-olefin is not necessary, and internal olefins containing 4 to 22 carbon atoms, or mixtures of isomers of linear or branched alkenes are also suitable for use. Non-limiting examples of suitable internal olefins include: 2-butene, 2-pentene, 2-hexene, 3-hexene, 2-heptene, 3-heptene, 2-octene, 3-octene, 4-octene, 2-nonene, 3-nonene, 4-nonene, 2-decene, 3-decene, 4-decene, 5-decene, etc, or mixtures of two or more of these compounds.

Acidic etherification catalysts suitable for use in the synthesis of the ether compound include, but are not limited to, acidic ionic exchange resins, such as DOWEX DR-2030 available from The Dow Chemical Company, clays, zeolites, sulfonated polystyrene beads, and acids immobilized on a heterogeneous surface, such as tetrafluoroethanesulfonic acid on silica beads, Bronsted acids such as triflic (trifluoromethanesulfonic) acid, methanesulfonic acid, or sulfuric acid, Lewis acids such as $BF_3$ and its derivatives (e.g., dihydrate or ether), and trimethylsilyl triflate. The ratio of catalyst to reactants is not critical and is generally adjusted so as to obtain a desired reaction rate. Preferably, the catalyst is at a temperature of between about 50 and 150 °C during the process in order to facilitate the etherification reaction.

The alkylating agent of preference is the haloalkyl compound of chloromethane for reasons of cost, safety profile (relative to stronger alkylating agents such as iodomethane and dimethyl sulfate), and it reacts more cleanly than dimethyl carbonate. With the preference noted, however, other alkylating agents include bromomethane, iodomethane, dimethyl sulfate, dimethyl carbonate, benzyl chloride, and chloroethane. Other "X" counterions can be created by controlled hydrolysis (e.g., the methyl sulfate anion formed in the reaction with dimethyl sulfate can be hydrolyzed to sulfate), or by ion exchange on an appropriate resin.

The contacting step of the process requires that the diamine of formula I and the alkylating agent are brought into contact with one another under ionizing conditions, i.e., under conditions sufficient to produce a cationic surfactant of formula II. Typically this contacting is performed in the liquid phase, using a solvent such as methanol and at elevated temperature and pressure, such as 50 to 150 °C and 50 to 200 psig. Following sufficient time for the reaction to occur (e.g., 2 to 20 hours), the reaction mixture is cooled and depressurized to ambient conditions, and then subjected to conventional workup. The formula II compound may optionally be further purified. Purification may be conducted using conventional techniques, such as extraction, filtration, chromatography, and/or crystallization.

When the cationic surfactant of the invention comprises a mixture of compounds of formula II, the composition of the mixture may be controlled by altering the contacting conditions, including alkyl chain length, reaction temperature, pH and reagent loadings. In this way the surfactant product characteristics and properties may be tailored to match the needs of the desired application.

The cationic surfactants of formula II may be used in a wide variety of compositions and applications where the presence of surfactants is desired or needed. By way of non-limiting example, the surfactants may be used as or in: laundry detergents, paint and coatings formulations, emulsion polymerization agents or formulations, household and industrial cleaners, agricultural formulations, latex formulations, environmental remediation agents, oilfield chemicals, enhanced oil recovery formulations, gas treating formulations, textile processing and finishing agents, pulp and paper processing agents, fragrance solubilization agents formulations, metal working fluids such as cutting fluids, personal care products (including skin and hair care products such as shampoos), and the like. The amount and composition of the compound of formula I to be used in these applications will vary depending on the application and the desired result and can be determined by a person of ordinary skill in the art without undue experimentation. Typically a composition that includes a compound of formula II as a surfactant will contain at least 0.01, more typically at least 0.05, even more typically at least 0.2 and still more typically 0.3, weight percent of the surfactant, based on the total weight of the composition. The maximum amount of formula II as a surfactant in a composition can vary widely and will typically be determined by such factors as economy and convenience, but typically the maximum amount does not exceed 20, more typically it does not exceed 10 and even more typically it does not exceed 5, weight percent of the surfactant, based on the total weight of the composition.

In a preferred embodiment, the surfactants of formula II are used in personal care products, particularly hair care products such as shampoos and conditioners. Such products impart excellent combability, softness and shine to human hair. The amount of the surfactant of formula II that should be used in a shampoo or conditioner composition can be easily determined by a person of ordinary skill in the art. By way of example, the amount is typically from about 0.01 % to 20 %, preferably 0.3 % to 5 %, by weight based on the total weight of the composition.

Shampoo and conditioner compositions may optionally contain other additives commonly used in such compositions including, for instance, one or more additives selected from: other cationic surfactants, nonionic surfactants, anionic surfactants, amphoteric surfactants, enzymes, solvents, hydrotropes, builders, thickening agents, chelating agents, perfume, dyes, opacifiers, optical brighteners, bleaching agents, and pH buffers.

When present, the amounts of such optional additives are preferably as follows: cationic surfactants in the range of 0.01% to 50%, preferably from 0.01% to 25%, more preferable, 1% to 20%; non ionic surfactants in the range of 0.01% to 20%, preferably from 0.01 to 15% more preferably from 0.5% to 10%; anionic surfactants in the range of 0.01% to 20%, preferably from 0.01 to 15% more preferably from 0.5% to 10%; amphoteric surfactants in the range of 0.01% to 20%, preferably from 0.01 to 15% more preferably from 0.5% to 10%; enzymes in the range of 0.0001% to 6%; solvents in the range of 0.01% to 20%, preferably from 0.01 to 15% more preferably from 0.5% to 10%; builders in the range of 1% to 60%; chelating agents in the range of 0.1% to 20%; and hydrotropes in the range of 0.1% to 15%, preferable from 0.5% to 10%.

*Amphoteric Surfactants*

In one embodiment the invention is a new class of amphoteric surfactants of formula III. These surfactants are of essentially the same structure as the cationic surfactants of formula II but without the associated X anion and with one of the R groups being negatively charged, i.e., R7 is a mono-anionic group of the formula $-(CH2)_mY$, where m is an integer from 0 to 4, and Y is a carboxylate, sulfonate, sulfate, phosphonate, or phosphate group. The amphoteric surfactants are made by a process comprising the step of contacting under ionizing conditions (i) a diamine of formula I, and (ii) an alkylating agent, such as sodium chloro-acetate, chloro-sulfonic acid or the like. These surfactants are used in a manner and in applications similar to that of the cationic surfactants.

*Nonionic Surfactants*

In one embodiment the invention is a new class of nonionic surfactants of formula IV. These surfactants are of essentially the same structure as the cationic surfactants of formula II but without the associated X anion and with one of the R groups replaced with an ionized oxygen atom. The nonionic surfactants are made by a process comprising the step of contacting under oxidizing conditions (i) a diamine of formula I, and (ii) an oxidizing agent, such as hydrogen peroxide. "Oxidizing conditions" include a temperature of 20 to 250, preferably 35 to 200 and more preferably 50 to 150, °C. Oxidizing conditions may also include a solvent to facilitate the reaction, e.g., water, alcohols, nitriles, halocarbons, N,N-dialkylamides, aliphatic esters and the like, or mixtures thereof.. The upper limit on the temperature is such as to avoid Hoffman degradation of the reaction product. Pressure is a function of the solvent and oxidizing agent. The optional solvent should be sufficiently polar to allow the intermediate mono-quat to remain in solution at the reaction temperature. Residence time is a function of the substrates and temperature. Typically, the reaction time is sufficient to completely convert the diamine, and this time is generally greater than one hour and can be as long as 24 hours or more. The process is usually performed without a catalyst. The nonionic surfactants are used in a manner and in applications similar to that of the cationic and/or amphoteric surfactants.

As noted above, the invention provides a new class of amine-containing, bicephlic surfactants that exhibit an improved spectrum of properties, allowing them to be used in a wide variety of applications. Such properties include high solubility in water, high tolerance to hard water, electrolytes, and caustic solutions, and, when used in cleaning compositions, excellent performance in hair care products.

The following examples are illustrative of the invention but are not intended to limit its scope. Unless otherwise indicated, the ratios, percentages, parts, and the like used herein are by weight.

EXAMPLES

*Example 1A: Preparation of $C_{12}$-diquat-TM (N,N,N,N',N',N'-hexamethyl-2-(2-dodecyloxy)-1,3-propanediaminium dichloride)*

Step 1: Preparation of $C_{12}$-diamine2 (N,N,N',N'-tetramethyl-2-(2-dodecyloxy)-1,3-propanediamine from 1,3-dichloropropan-2-yloxydodecanes

A $N_2$-purged 450-mL Parr reactor equipped with a propeller-type impeller and removable glass liner is charged with 17.7 grams (g) of $N_2$-sparged 1,3-dichloropropan-2-yloxydodecanes (59.5 mmol) via syringe. The reactor is then loaded *via* cannula with 115.8 g dimethylamine solution (40 % in water, Aldrich; 1,028 mmol; 17.3 eq.) which is not deaerated. The reactor is sealed, and agitation and heating initiated. The temperature reaches a maximum of 119 °C within 100 min and then is stabilized between 106-108 °C for 16 hours. The reactor is then allowed to cool to 80 °C and the pressure is slowly released *via* a gas disperser into a large amount of 1 N HCl. The reactor is opened at room temperature, revealing a cloudy-white lower layer and a very thin, dark upper layer. These are separated by addition of 100 mL diethyl ether and the aqueous layer is washed twice with 75 mL ether. The combined organic fractions are dried over anhydrous $MgSO_4$, filtered, and reduced by rotary evaporation to an oil. This is then distilled at 0.6-0.9 mmHg and

129-136 °C using a short-path micro-distillation apparatus. Yield of clear, colorless, slightly viscous oil: 14.44 g (FW = 314.55, 77 %). $^{1}$H NMR ($CHCl_3$), ppm: (δ) 3.3-3.75 (m, 2.3 H); 2.2-2.4 (m, with two strong non-equal peaks, 13 H); 1.5 (br m), 1.24 (br s), 1.12 (d, 6 Hz), 0.87 (m); total alkyl integral (0.8-1.6) 24 H. $^{13}$C NMR ($CHCl_3$), ppm: (δ) 79.2, 63.3, 63.1, 46.7, 37.4, 32.1, 30.0, 29.5, 25.9, 22.8, 20.6, 14.3 (minor peaks also seen at 44.2, 43.9, 43.8, 26.9, 25.6). Calcd. for $C_{19}H_{42}N_2O$ (%): C, 72.55; H, 13.46; N, 8.91; O, 5.09; Cl, 0. Found: C, 71.36; H, 13.14; N, 7.92; O, 4.58; CL, 0.38.

Step 2: Preparation of $C_{12}$-diquat-TM (1)(N,N,N,N',N',N'-hexamethyl-2-(2-dodecyloxy)-1,3-propanediaminium dichloride) from $C_{12}$ Diamine (N,N,N',N'-tetramethyl-2-(2-dodecyloxy)-1,3-propanediamine)

A $N_2$-purged 450-mL Parr reactor equipped with a propeller-type impeller and removable glass liner is charged with a mixture of 40 mL methanol and 10 mL water and stirring initiated. The reactor is then sealed and placed under nitrogen by 10 pressurizations to 32 psi followed by venting to atmospheric pressure. After three pressurizations to 30 psi with chloromethane followed by venting to atmospheric pressure, the reactor is allowed to fill with chloromethane to a pressure of 50 psi at 30 °C at which point the flow of chloromethane is stopped. After 5 minutes the reactor temperature falls to 25 °C and the pressure to 40-45 psi. Through a septum port are added 8.01 g $C_{12}$-diamine2 (25.5 mmol). The reactor is then heated to 89-95 °C for 17.5 hours during which time the pressure rises to 90 psi before falling to 75-80 psi. After cooling and venting the reactor, the reaction mixture, a brownish oily liquid, is reduced to a thick tar by rotary evaporation. Ethanol (50 mL) is added and removed by rotary evaporation twice. Then ethanol (80 mL) is added and the resulting solid filtered, washed with ethanol, and dried to a pale blue powder (2.42 g). The filtrate is concentrated to a dark oil, mixed with 80 mL diethyl ether, and agitated overnight. The resulting wet cake is centrifuged, and the supernatant discarded. More diethyl ether is added, and the blue solid filtered and washed with more diethyl ether. After drying in air, the solid turns to a tarry green wax. The sample contains 16.6 wt % inorganic chloride by $AgNO_3$ titration (theoretical 17.1) and 5 % water by Karl Fischer titration. Yield 7.67 g (FW = 415.52, 69 %, corrected for water). Proton NMR spectrum consists of broad, overlapping peaks. $^{1}$H NMR ($CD_3OD$), ppm: (δ) 3.69 (br m), 3.30 (br s), 2.98 (br s), 1.69 (br m), 1.26 (br s), 1.15 (m), 0.94 (br s), 0.86 (br s). The ratio of total integrals for the region 4.1-2.6 ppm to that for the region 2.0-0.6 ppm = 22.4:24 (theor. 24:24). NMR spectra are collected on a Varian Inova 400 MHz spectrometer. Elemental analyses are provided by Quantitative Technologies, Inc., Whitehouse, NJ.

*Example 1B: Preparation of $C_{16}$-diquat-TM (N,N,N,N',N',N'-hexamethyl-2-(2-hexadecyloxy)-1,3-propanediaminium dichloride)*

Step 1: Preparation of N,N,N',N'-tetramethyl-2-(2-hexadecyloxy)-1,3-propanediamine ($C_{16}$-diamine2) from 1,3-dichloropropan-2-yloxyhexadecanes

A $N_2$-purged 450-mL Parr reactor equipped with a propeller-type impeller and removable glass liner is charged with 26.80 g of 1,3-dichloropropan-2-yloxyhexadecanes (75.8 mmol) via syringe and 130.35 g dimethylamine solution (40 % in water, Aldrich; 1.157 mmol; 15.3 eq.) *via* cannula. The reactor is sealed under 10 psi $N_2$, and heated to 105-116 °C (90-110 psi) for 19 hours. After cooling to 30 °C, the reactor is allowed to vent and is then purged with flowing $N_2$ for about 75 min. The reactor contents are poured into a separatory funnel with 200 mL water and 100 mL hexanes. The aqueous fraction is washed twice with 100 mL hexanes and the combined organic fractions are dried over anhydrous $MgSO_4$ and reduced by rotary evaporation to a clear yellow oil. The product is distilled using a short-path jacketed column at 0.27-0.34 mmHg and 159-168 °C. Yield of clear, colorless, slightly viscous oil: 24.822 g (FW = 370.39, 88 %). $^{1}$H NMR ($CHCl_3$), ppm: (δ) 3.3-3.8 (m, 2.9 H); 2.2-2.5 (m, with two strong non-equal peaks, 13 H); 1.5 (br m), 1.26 (br s), 1.14 (d, 6 Hz), 0.89 (m); total alkyl integral (0.8-1.6) 32 H.

Step 2: Preparation of N,N,N,N',N',N'-hexamethyl-2-(2-hexadecyloxy)-1,3-propanediaminium dichloride ($C_{16}$-diquat-TM) from N,N,N',N'-tetramethyl-2-(2-hexadecyloxy)-1,3-propanediamine) ($C_{16}$-diamine2)

A 450-mL Parr reactor with an impeller removed and thermocouple housing sheathed by a glass NMR tube section containing mineral oil and sealed with PTFE tape and with removable glass liner containing a magnetic stirbar is sealed and purged with $N_2$. To the reactor is added 5 mL water and a $N_2$-sparged mixture of 15.42 g $C_{16}$-diamine2 (41.6 mmol) and 55.70 g methanol. The reactor is rapidly pressurized with chloromethane (Aldrich, 99.5 %) and depressurized for three cycles, then allowed to equilibrate with the tank of chloromethane for 16 minutes after which the tank is closed and the reactor heated to 86-7 °C for 19 hours. After cooling to 36 °C the reactor is vented and then purged with $N_2$ flow for 6 minutes. The resulting clear, green solution is reduced to an amber oil by rotary evaporation. To the soapy cake is then added 50 mL ethanol, which is removed by rotary evaporation, followed by a second ethanol charge of 100 mL, which is also similarly removed. A mixture of the resulting solid in ethyl ether and hexanes could not be filtered, so it is allowed to dry in air and then by vacuum drying (42 °C, overnight). Yield of waxy solid: 18.41 g. Proton

NMR spectrum consisted of broad, overlapping peaks. $^1H$ NMR ($CD_3OD$), ppm: (δ) 3.5-3.8 (br m), 3.3 (br s), 3.0 (br s), 1.26 (br s), 1.15 (m), 0.94 (br s), 0.87 (m). The ratio of total integrals for the region 4.1-2.9 ppm to that for the region 2.0-0.6 ppm = 19.6:32 (theor. 24:32). $^{13}C$ NMR ($CD_3OD$), ppm: (δ) 74.4, 70.0, 69.6, 69.2, 66.8, 38.0, 33.1, 30.5-31.1, 26.4-26.8, 23.7, 20.8, 19.9, 18.8, 14.5 (with many minor peaks). Calcd. for $C_{25}H_{56}Cl_2N_2O$ (%): C, 63.67; H, 11.97; N, 5.94; Cl, 15.03. Found: C, 60.68; H, 11.73; N, 4.41; Cl, 15.29. Inorganic chloride ($AgNO_3$ titration): 12.6 wt % (theoretical 15.0) and 3.3 % water by Karl Fischer titration. Transition metal contents (ICP): Fe, 0.31 %; Cr, 0.17 %; Ni, 0.04 %.

*Example 1C: Preparation of C12 Dibetaine (2,2'-(dodecan-2-yloxypropane-1,3-diyl)bis)(dimethylammonionediyl)diacetate)*

A 250-mL round bottom flask was charged with 24.6 g (0.211 mol) of sodium chloroacetate, 4.58 g (0.043 mol) of sodium carbonate. 26.77 g (0.0852 mol) of N,N,N',N'-tetramethyl-2-dodecyloxypropyl-1,3-diamine, 90 mL of methanol, and 10 mL of water. After heating at reflux for two days, the mixture was cooled in an ice bath, filtered, and the filtercake rinsed with methanol. The filtrate was concentrated to afford 47.8 g of a clear oil containing 2,2'-(dodecan-2-yloxypropane-1,3-diyl)bis)(dimethylammonionediyl)diacetate with 5 wt% sodium chloride (ion selective electrode). $^1H$ NMR ($D_2O$), ppm: (δ) 4.6-4.9 (br m), 3.9 (br, m), 3.3 (br s), 2.4 (br s), 2.25 (s), 1.2-1.8 (br s), 0.9 (s). $^{13}C$ NMR ($D_2O$), ppm: (δ) 170.4, 170.3, 170.2, 170.1, 75.0, 73.4, 67.2, 67.4, 67.8, 68.3, 54.6, 54.9, 55.0, 55.5, 55.8, 16 to 51, multiple peaks. Mass Spec: $C_{23}H_{46}N_2O_5$, Exact Mass 430.340672.

*Example 1D: Preparation of C16 N-Oxide (hexadecan-2-yloxy-N,N,N',N'-tetramethylpropane-1,3-diamine dioxide)*

A 100 mL three-neck round-bottom flask was charged with 8.91 g C16-diamine2 (24.1 mmol), 27 mL distilled water, and 37 mg disodium EDTA dihydrate (0.10 mmol) (to complex any transition metals present). The flask was flow-purged with nitrogen for about 40 min, then left under static pressure with an oil bubbler. An addition funnel was charged with 5.26 g 35 wt % hydrogen peroxide (Acros Organics, stabilized, 54.1 mmol, 2.25 eq.). The mixture was heated by heating mantle to 61 °C, whereupon addition of the peroxide was initiated. This was done slowly, one or two drops at a time, over a period of 1 h, with the temperature kept between 59-65 °C. The temperature was raised to 72-76 °C and the mixture stirred for another 3 h. Within 1 h, the hazy emulsion had become clear. The mixture was allowed to cool to ambient temperature and stir overnight. Using peroxide test strips, the residual hydrogen peroxide was monitored as sodium bisulfite (Aldrich ACS grade) was added in small portions. Each addition caused a slight increase in temperature, ending at 32.5 °C. After addition of 1.15 g sodium bisulfite (10.8 mmol), no peroxide was detected, and a similar reading was obtained after a further 10 min stirring, generating a 40.3 g clear, colorless, somewhat soapy liquid. (The solution turned yellow within a few days.) A portion of the solution was dried at 52 °C in a vacuum oven overnight with nitrogen flow, yielding 28.8 wt % of a yellow, viscous oil/wax (calc. 27 wt % solids). Based on the consumption of bisulfite, the conversion of amine to amine N-oxide was estimated to be 90 %. $^1H$ NMR (δ), ppm: 4.4 (br s, 0.3 H), 3.5-3.9 (m, 3.4 H), 3.3-3.4 (m, 7.3 H), 2.74 (s), 2.69 (s), [2.5-3.1: 3.9 H], 1.3 (br s), 0.92 (br s), [0.7-1.9: 32 H]. $^{13}C\{^1H\}$ (δ), ppm: 74.8, 73.2, 72.9, 71.1, 58-60 (br), 37.0, 32.2, 30.2 (br), 25.4, 19.0, 14.0. No peaks were observed in the region of the proton NMR spectrum associated with neutral methylamine groups (2-2.5 ppm), but a small, broad absorbance at 43-45 ppm in the $^{13}C$ spectrum may indicate unreacted amine. The ratio of the summed proton NMR integrals for peaks not associated with the alkyl chain to that for the chain, 15:32, is close to that expected for the starting material and product (17:32).

*Example 2: Inventive and Comparative Formulations in Hair Care Applications*

Materials used in formulations of the invention and comparative formulations are listed in Table 1.

Table 1

| Materials | | |
|---|---|---|
| **Tradename** | **Chemical or CTFA (INCI) name** | **Manufacturer** |
| Procol CS 20D | Cetearyl alcohol (and) ceteareth-20 | Protameen Chemicals |
| Dow Corning 345 Fluid | Cyclopentasiloxane (and) cyclohexasiloxane | Dow Corning Corporation |
| Jeechem S-13 | Stearamidopropyl dimethylamine | Jeen International Corporation |
| Macquat BTMC 85 | Behentrimonium chloride | Mason Chemicals Company |
| Stearyl alcohol | Stearyl alcohol | Tokyo Kasei Co. |
| Glucam E-10 | Methyl gluceth-10 | Noveon, Inc. |

(continued)

| Materials | | |
|---|---|---|
| **Tradename** | **Chemical or CTFA (INCI) name** | **Manufacturer** |
| Glydant | DMDM Hydantoin | Lonza, Inc. |
| Jeequat SDQ-85 | Stearalkonium chloride | Jeen International Corporation |
| UCARE JR-400 | Polyquaternium-10 | The Dow Chemical Company |
| Standapol ES-2 | Sodium Laurel Sulfate | Cognis Corporation |
| Velvetex CDC | Disodium Cocoamphodiacetate | Cognis Corporation |
| Cetyl Alcohol | Lanette 16 | Cognis GmbH |
| Cetrimonium Chloride | Protaquat CT-29 | Protameen Chemicals |
| Hydroxyethyl Cellulose | Cellosize PCG-10 | The Dow Chemical Company |
| Hydrogenated Vegetable Oil | Lipo SS | Lipo Chemicals |
| Panthenol | DL-Panthenol | Protameen Chemicals |

*Example 2A: Rinse-off Conditioner Formulation of the Invention Containing Novel Diquat Material (C1) and Comparative Formulation Containing Behentrimonium Chloride*

Rinse-off hair conditioner formulation (C1) containing diquat (1) of the invention and a similar comparative formulation (C2) containing cosmetic monoquat behentrimonium chloride (BTC) instead of diquat (1) are presented in Table 2. The amounts of ingredients in Table 2 are expressed in percents on a weight/weight basis (wt. %). Note that the diquat (1) is incorporated in formulation (C1) at 0.5 wt. % level whereas formulation (C2) contained 2 wt. % (five times more) of BTC monoquat.

Table 2

| Rinse-Off Conditioner Formulations | | | |
|---|---|---|---|
| **INCI Name** | **Trade Name** | **(C1)** | **(C2)** |
| **Phase A** | | | |
| Stearamidopropyl Dimethylamine | Jeechem S-13 | 0.8 | 0.8 |
| Stearyl Alcohol | Stearyl Alcohol | 1.5 | 1.5 |
| Cetearyl Alcohol (and) Ceteareth-20 | Procol CS 20 D | 3.0 | 3.0 |
| Behentrimonium chloride | Macquat BTMC 85 | | 2.0 |
| **Phase B** | | | |
| N/A | C12-Diquat (1) | 0.5 | |
| Water (Aqua) | DI Water | 25.0 | 25.0 |
| **Phase C** | | | |
| DI Water | DI Water | 66.8 | 65.3 |
| Methyl Gluceth -10 | Glucam E-10 | 1.0 | 1.0 |
| **Phase D** | | | |
| Cyclopentasiloxane (and) Cyclohexasiloxane | DC 345 | 1.0 | 1.0 |
| DMDM Hydantoin | Glydant | 0.4 | 0.4 |
| **Total** | | 100.0 | 100.0 |

C1 is formulated by combining the Phase A components and then mixing and heating the mixture to 75°C under stirring. In a separate reaction vessel, Phase C ingredients are combined, mixed and heated to 75°C under stirring.

Phase C is added to Phase A at 75°C under stirring. The reaction mixture or batch is cooled at room temperature while stirring. Glydant (Phase C, preservative) is added at 35°C. The pH of the formulation is adjusted to 4-5 using 50% citric acid as needed. Phase B pre-mix is made by adding the diquat material at room temperature to water. Pre-mix B is then added slowly to the batch and mixed until the batch is uniform.

C2 is formulated by combining Phase A and B components and then mixing and heating the mixture to 75°C under stirring to form a batch. In a separate reaction vessel, Phase C ingredients are combined, mixed and heated to 75°C under stirring. Phase C is added to the batch at 75°C under stirring at 470 rpm. The reaction mixture is cooled at room temperature while stirring. Glydant (Phase C, preservative) is added at 35°C. The pH of the formulation is adjusted to 4-5 using 50% citric acid as needed.

*Example 2B: Shampoo Formulation of the Invention Containing Novel Diquat Material (2) and Comparative Shampoo Formulations Containing Benchmark Quaternary Materials*

Shampoo formulations (S1) containing diquat of the invention (2) and comparative formulations (S2)-(S4) containing cosmetic monoquats behentrimonium chloride and stearalkonium chloride, and Polyquaternium-10 (polymer JR-400) instead of diquats are presented in Table 3. The amounts of ingredients in Table 3 are expressed in percents on a weight/weight basis (wt. %). All quaternary materials are incorporated in formulations (S1)-(S4) at 0.5 wt. % level.

Table 3

| Shampoo Formulations | | | | | |
|---|---|---|---|---|---|
| **INCI Name** | **Trade Name** | **(S1)** | **(S2)** | **(S3)** | **(S4)** |
| Sodium Laureth Sulfate, 25.5% | Standapol ES-2 | 60.8 | 60.8 | 60.8 | 60.8 |
| Disodium Cocoamphodiacetate, 38.5% | Velvetex CDC | 6.9 | 6.9 | 6.9 | 6.9 |
| N/A | C16-Diquat (2) | 0.5 | | | |
| Behentrimonium Chloride | Macquat BTMC 85 | | 0.5 | | |
| Stearalkonium Chloride | Jeequat SDQ-85 | | | 0.5 | |
| Polyquaternium-10 | UCARE JR-400, 2% | | | | 25.0 |
| DI Water | DI Water | 29.2 | 29.2 | 29.2 | 4.7 |
| Citric acid, 10% | Citric Acid | 2.2 | 2.2 | 2.2 | 2.2 |
| DMDM Hydantoin, 50% | Glydant | 0.4 | 0.4 | 0.4 | 0.4 |
| **Total** | | 100.0 | 100.0 | 100.0 | 100.0 |

The protocol for formulating (S1)-(S3) is as follows;

1. Mix Standapol ES-2, Velvetex CDC, and DI water. Stir at high speed until uniform.

2. With a water bath, increase temperature to 70°C.

3. Add monoquat material and mix for 30 minutes.

4. After 30 minutes, slowly cool to room temperature.

5. Add 10% citric acid solution to formulation and stir for 10 minutes.

6. Add Glydant, and mix thoroughly.

7. Add water, *q. s.*

The protocol for formulating (S4) is as follows;

1. Mix Standapol ES-2, Velvetex CDC, and DI water. Stir at high speed until uniform.

2. With stirring at high speed, slowly add 2% JR-400 solution, and continue stirring at high speed until well mixed.

3. With a water bath, increase temperature to 70°C.

4. After 30 minutes, slowly cool to room temperature.

5. Add 10% citric acid solution to formulation and stir for 10 minutes.

6. Add Glydant, and mix thoroughly.

7. Add water, *q. s.*

*Example 2C: Leave-on Conditioner Formulations of the Invention Containing Novel Diquat Materials (C3) and (C4)*

Leave-on hair conditioner formulations (C3) and (C4) containing diquats (1) and (2) of the invention respectively are presented in Table 4A. The amounts of ingredients in Table 4A are expressed in percents on a weight/weight basis (wt. %).

Table 4A

| Leave-On Conditioner Formulations | | | |
|---|---|---|---|
| **INCI Name** | **Trade Name** | **(C3)** | **(C4)** |
| **Phase A** | | | |
| Water | Water | 66.5 | 66.5 |
| Hydroxyethyl Cellulose | Cellosize PCG 10 | 0.5 | 0.5 |
| Cetrimonium Chloride | Protaquat CT-29, 29% | | |
| Hydrogentated Vegetable oil | Lipo SS | 2.0 | 2.0 |
| Panthenol | Panthenol | 0.1 | 0.1 |
| **Phase B** | | | |
| Cetyl Alcohol | Lanette 16 | 2.0 | 2.0 |
| Stearyl Alcohol | Stearyl Alcohol | 3.0 | 3.0 |
| **Phase C** | | | |
| Water | Water | 25.0 | 25.0 |
| N/A | C12-Diquat (1) | 0.5 | |
| N/A | C16-Diquat (2) | | 0.5 |
| **Total** | | 100.0 | 100.0 |

The formulations are prepared according to the following protocol:

1. Cellosize PCG-10 is sprinkled into rapidly agitating room temperature water. Once uniform the batch temperature is brought up to 75-80°C.

2. In a separate container fatty alcohols are heated to the same temperature, mixed until uniformed (Phase B) and added to the batch. The batch is mixed until uniform.

3. Vegetable oil is mixed into the batch until the batch is uniform.

4. The batch is cooled to 40°C and Panthenol is added.

5. In a separate container experimental di-quat material is dispersed in water (Phase C) and added to the pre-mix under stirring.

6. The pH is adjusted to 4.5-5.5 with citric acid.

7. Glydant preservative is added and the batch is mixed until uniform.

8. Water is added, *q.s.*

*Example 3: Subjective Evaluation of Wet hair Properties (Rinse-off Conditioners)* Hair Treatment Procedure

Pre-washed and pre-hydrated ten inch long hair tresses of European single-bleached hair available from International Hair Importers and Products Inc. (Floral Park, NY) are treated with the rinse-off conditioner formulations. Conditioner product (0.1 g) is applied to each tress of hair. The product is worked into the hair for 1 minute and then rinsed off under running tap water at 38°C and 0.4 gal./min water flow for 1 minute. The tresses are hanged to dry overnight.

Evaluation Procedure

On the next day five expert panelists trained to evaluate performance of cosmetic products on hair are asked to evaluate combability and feel of dry hair tresses. Each panelist evaluates two pairs of tresses. Each pair has one tress treated with the composition of the invention (C1) versus one treated with the comparative composition (C2) containing 2 wt. % BTC. The panelists are asked to pick one tress that combed easier and felt smoother/softer.

Statistical Data Analysis

Additionally, the subjective evaluations are statistically analyzed to identify differences at above 89 % confidence level. The results are reported in Table 4B.

Table 4B

| Subjective Panel Evaluation against Comparative Rinse-Off Conditioner Formula (C2) with BTC | | |
|---|---|---|
| **Formulation** | **Dry Comb** | **Dry Feel** |
| (C1) | 9/10 (+) | 9/10 (+) |
| + means that the inventive sample is statistically superior to the control sample (minimum of 8 out of 10 the inventive sample is rated superior). | | |

The data reported in Table 4 shows that the rinse-off conditioner (C1) containing only 0.5 % of diquat material of the invention (1) is found superior to the comparative formulation (C2) containing 2 wt.% BTC in dry comb and feel. The result is statistically significant at the chosen confidence level. This example shows that the diquat material of the invention functions as an efficient conditioning agent in rinse-off hair conditioner formulations. Used at one-fourth of the monoquat level, it outperformed the leading conditioning monoquat BTC, typically used in this type of personal care applications.

*Example 4: Objective wet Comb-ability Measured Using the Dia-stron Miniature Tester* (*Shampoos*)

Hair Treatment Procedure

Pre-washed and pre-hydrated eight inch long hair tresses of European single-bleached hair available from International Hair Importers and Products Inc. (Floral Park, NY) are treated with the shampoo formulations. Conditioner product (0.5 g) is applied to each tress of hair. The product is worked into the hair for 1 minute and then rinsed off under running tap water at 38°C and 0.4 gal/min water flow for 1 minute.

Evaluation Procedure

All measurements are carried out using 8 inch long tresses of European bleached hair that weighed about 4 g. The wet combing work (WCW) is measured by using the load cell of an Instron Tensile Tester when a comb is pulled

through a wet tress of European single-bleached hair available from International Hair Importers and Products Inc. The wet combability of a shampoo formulation is calculated as follows in terms of the wet combing work done (WCWD), % (percent) reduction of a hair tress treated with formulation (S1) of the invention or comparative formulations (S2)-(S4), as compared to a hair tress treated with a base formulation comprising the same ingredients as formulations (S1)-(S4) but no quaternary monoquats or polyquats:

$$\%\text{WCWD Reduction} = [(\text{WCWD}_{\textit{base}} - \text{WCWD}_{\textit{treated}})]/\text{WCWD}_{\textit{base}}] \times 100$$

where *base* means that the hair tress is treated with the base formulation without monoquats/polyquats, and *treated* means that the hair tress is treated with the shampoo formulation (S1)-(S4).

Table 5

| Wet Combability | |
|---|---|
| **Formulation** | **WCWD % reduction** |
| (S1) | 54% |
| (S2) | 33% |
| (S3) | 0% |
| (S4) | 39% |

Results in Table 5 show that shampoo formulations containing di-quat derivatives of the present invention have better conditioning performance compared to commercially available cosmetic monoquats (Formulations (S2) and (S3)) and Polyquaternium-10 UCARE JR-400 (Formulation (S4)). Specifically, hair treatment with formulation (S1) results in a greater reduction in wet combing work compared to treating hair with comparative formulations (S2)-(S4).

*Example 5: Adjuvants in Glyphosate Formulations for Agricultural Applications*

Cationic surfactants described in this invention are added to RODEO formulations (glyphosate IPA 480 g ae/L, no built-in adjuvants), and their performance is evaluated against commercial formulations with built-in adjuvants: Durango DMA (glyphosate DMA 480 g ae/L), and Roundup WeatherMax (glyphosate K+ 540 g ae/L).

Cationic surfactants described in this invention are added to the spray solution at a concentration equivalent to the amount of surfactant typically included in glyphosate formulations (0.18% v/v surfactant at 800 g ae/ha glyphosate in 140 l/ha total spray volume). The adjuvant level is titrated as to directly compare with the performance of the standards.

In this example, the following species are tested: Wild Poinsetta, Morning glory, Barnyard grass, Velvetleaf, Wild Oats, Quackgrass, Common Lambsquarters, Sicklepod. Dose Rates (g ae/ha) 0, 100, 200, 400, 600 and 800; Spray Volume: 140 l/ha = 15 gpa; each test is repeated three times. Percent control is averaged across tested species and at dose rate of 400 G/ha:

Table 6

| **Formulation** | **% Control** |
|---|---|
| RODEO | 41 |
| RODEO+C16 Diquat | 73 |
| RODEO+C12 Diamine | 68 |
| DURANGO | 67 |
| WEATHERMAX | 74 |

*Example 6: Catalysts in Epoxy Applications*

Anhydride-cured epoxy system is used in this Example. Materials used in the formulations of the invention are: Epoxy D.E.R. 383, anhydride curing agent ECA 100 (blend of methyltetrahydrophthalic anhydride and tetrahydrophthalic anhydride), and amine based catalyst. Catal yst load is 1 wt%. $C_{12}$ Diamine and $C_{12}$ Diquat are compared to common catalysts used in epoxy chemistry, benzyldimethylamine (BDMA), and benzyltriethylammonium chloride (BTEAC), respectively.

$C_{12}$ diamine catalyst dissolved fast in epoxy/anhydride mixture. C12 Diquat is heated at 60°C overnight in anhydride for full dissolution, similar to BTEAC.

DSC screen is used to monitor reaction, and to give a relative comparison of the rate of catalysis between compared catalysts. Both the $C_{12}$ Diamine and the $C_{12}$ Diquat worked well as catalysts.

The DSC exotherms are slightly shifted to the higher temperature compared to BDMA or BTEAC at 1 wt% catalyst loading, but are expected to overlap if used at the same mol% of catalyst as the inventive materials have higher molecular weights, and thus have lower mole percent load at the same weight percent load.

Tested catalyst show good catalytic activity for epoxy-anhydride curing, offering lower volatility and opportunity to affect properties of final coatings via alkyl chain modification, e.g. hydrophobic surface modification.

## Claims

1. A diamine on formula I.

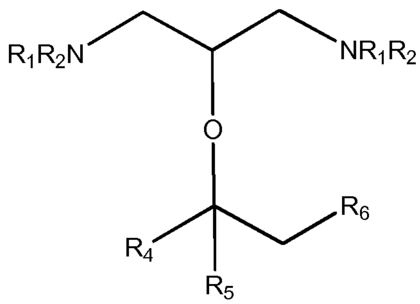

(I)

in which $R_1$ and $R_2$ are methyl, ethyl or butyl groups, or may be joined with one another to form a ring of 3-9 carbon atoms;

$R_4$ is hydrogen or a $C_1$-$C_8$ alkyl group;
$R_5$ is a $C_1$-$C_{10}$ linear alkyl group;
$R_6$ is a linear $C_2$-$C_{20}$ alkyl group;
$R_5$ and $R_6$ may be joined with one another to form a ring of 3-10 carbon atoms;
with the proviso that the combined carbon atom count of $R_4$, $R_5$, $R_6$ and the central carbon atom is 4 to 22.

2. A cationic surfactant of formula II:

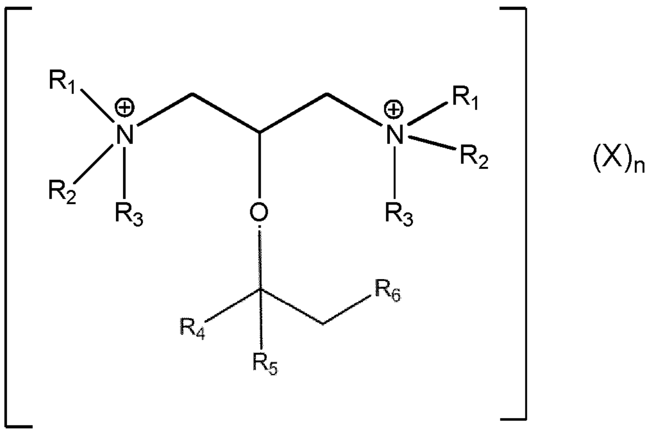

(II)

in which $R_1$ and $R_2$ are $C_1$-$C_4$ linear or branched alkyl groups, or may be joined with one another to form a ring of 3-9 carbon atoms;

$R_3$ is a $C_1$-$C_{20}$ linear or branched alkyl or arylalkyl group;
$R_4$ is hydrogen or a $C_1$-$C_8$ alkyl group;

$R_5$ is a $C_1$-$C_{10}$ linear alkyl group;
$R_6$ is a linear $C_2$-$C_{20}$ alkyl group;
$R_5$ and $R_6$ may be joined with one another to form a ring of 3-10 carbons;
with the proviso that the combined carbon atom count of $R_4$, $R_5$, $R_6$ and the central carbon atom is 4 to 22;
X is a mono-or di-anion of at least one of halide, bicarbonate, carbonate, sulfate, methyl sulfate and bisulfate; and
n is an integer such that the total anionic charge provided by $X_n$ is 2.

3. The cationic surfactant of Claim 2 in which $R_4$ is hydrogen.

4. An amphoteric surfactant of formula III:

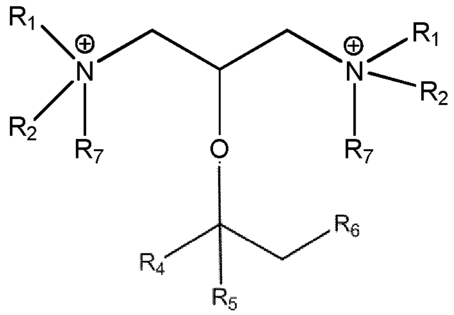

(III)

in which $R_1$ and $R_2$ are $C_1$-$C_4$ linear or branched alkyl groups, or may be joined to form a ring of 3-10 carbon atoms;

$R_4$ is hydrogen or a $C_1$-$C_8$ alkyl group;
$R_5$ is a $C_1$-$C_{10}$ linear alkyl group;
$R_6$ is a linear $C_2$-$C_{20}$ alkyl group;
$R_5$ and $R_6$ may be joined with one another to form a ring of 3-10 carbon atoms;
R7 is a mono-anionic group of the formula $-(CH2)_mY$, where m is an integer from 0 to 4, and Y is a carboxylate, sulfonate, sulfate, phosphonate, or phosphate group;
with the proviso that the combined carbon atom count of $R_4$, $R_5$, $R_6$ and the central carbon atom is 4 to 22;

5. The amphoteric surfactant of Claim 4 in which $R_4$ is hydrogen.

6. A nonionic surfactant of formula IV:

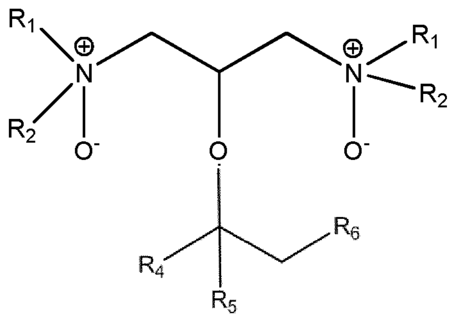

(IV)

in which $R_1$ and $R_2$ are $C_1$-$C_4$ linear or branched alkyl groups, or may be joined to form a ring of 3-10 carbon atoms;

$R_4$ is hydrogen or a $C_1$-$C_8$ alkyl group;
$R_5$ is a $C_1$-$C_{10}$ linear alkyl group;
$R_6$ is a linear $C_2$-$C_{20}$ alkyl group;
$R_5$ and $R_6$ may be joined with one another to form a ring of 3-10 carbon atoms;
with the proviso that the combined carbon atom count of $R_4$, $R_5$, $R_6$ and the central carbon atom is 4 to 22;

**7.** The nonionic surfactant of Claim 6 in which $R_4$ is hydrogen.

**8.** A composition comprising the cationic surfactant of Claim 2.

**9.** The composition of Claim 8 in the form of a laundry detergent, paint, coating, emulsion polymerization agent, household or industrial cleaner, agricultural formulation, latex formulation, environmental remediation agent, oilfield chemical, enhanced oil recovery formulation, gas treating formulation, textile processing, finishing or sizing agent, pulp or paper processing agent, fragrance solubilization agent or formulation, metal working fluid or personal care product.

**10.** The composition of Claim 8 in the form of a hair shampoo or conditioner.

**11.** A method of treating hair, the method comprising applying a composition of Claim 10.

**12.** A composition comprising the amphoteric surfactant of Claim 4.

**13.** The composition of Claim 12 in the form of a laundry detergent, paint, coating, emulsion polymerization agent, household or industrial cleaner, agricultural formulation, latex formulation, environmental remediation agent, oilfield chemical, enhanced oil recovery formulation, gas treating formulation, textile processing, finishing or sizing agent, pulp or paper processing agent, fragrance solubilization agent or formulation, metal working fluid or personal care product.

**14.** A composition comprising the nonionic surfactant of Claim 6.

**15.** The composition of Claim 14 in the form of a laundry detergent, paint, coating, emulsion polymerization agent, household or industrial cleaner, agricultural formulation, latex formulation, environmental remediation agent, oilfield chemical, enhanced oil recovery formulation, gas treating formulation, textile processing, finishing or sizing agent, pulp or paper processing agent, fragrance solubilization agent or formulation, metal working fluid or personal care product.

## Patentansprüche

**1.** Ein Diamin der Formel I:

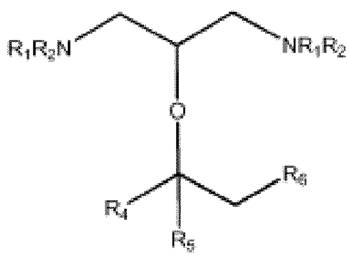

(I)

wobei $R_1$ und $R_2$ Methyl-, Ethyl- oder Butylgruppen sind oder miteinander verbunden werden können, um einen Ring von 3-9 Kohlenstoffatomen zu bilden;

$R_4$ Wasserstoff oder eine $C_1$-$C_8$-Alkylgruppe ist;
$R_5$ eine lineare $C_1$-$C_{10}$-Alkylgruppe ist;
$R_6$ eine lineare $C_2$-$C_{20}$-Alkylgruppe ist;
$R_5$ und $R_6$ miteinander verbunden werden können, um einen Ring von 3-10 Kohlenstoffatomen zu bilden;
mit der Maßgabe, dass die kombinierte Zahl von Kohlenstoffatomen $R_4$, $R_5$, $R_6$ und der zentralen Kohlenstoffatome 4 bis 22 beträgt.

**2.** Ein kationisches Tensid der Formel II:

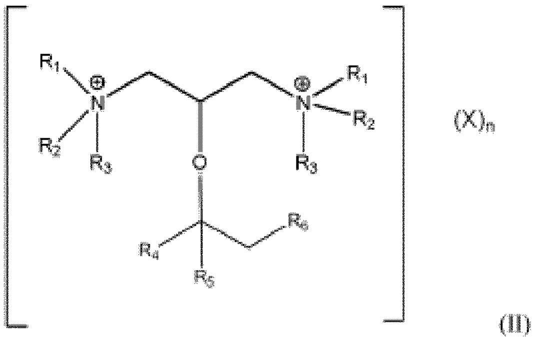

(II)

wobei $R_1$ und $R_2$ lineare oder verzweigte $C_1$-$C_4$-Alkylgruppen sind, oder miteinander verbunden werden können, um einen Ring von 3-9 Kohlenstoffatomen zu bilden; $R_3$ eine lineare oder verzweigte $C_1$-$C_{20}$-Alkyl- oder Arylalkylgruppe ist;

$R_4$ Wasserstoff oder eine $C_1$-$C_8$-Alkylgruppe ist;
$R_5$ eine lineare $C_1$-$C_{10}$-Alkylgruppe ist;
$R_6$ eine lineare $C_2$-$C_{20}$-Alkylgruppe ist;
$R_5$ und $R_6$ miteinander verbunden werden können, um einen Ring von 3-10 Kohlenstoffen zu bilden;
mit der Maßgabe, dass die kombinierte Zahl von Kohlenstoffatomen $R_4$, $R_5$, $R_6$ und der zentralen Kohlenstoffatome 4 bis 22 beträgt;
X ein Mono- oder Dianion von mindestens einem von Halogenid, Bicarbonat, Carbonat, Sulfat, Methylsulfat und Bisulfat ist; und
n eine ganze Zahl ist, sodass die anionische Gesamtladung, bereitgestellt durch $X_n$, 2 ist.

**3.** Kationisches Tensid gemäß Anspruch 2, wobei $R_4$ Wasserstoff ist.

**4.** Ein amphoteres Tensid der Formel III:

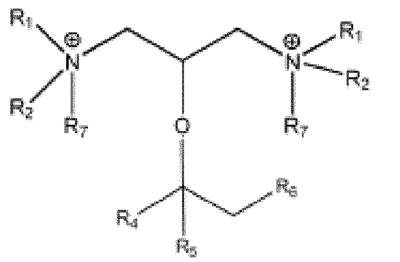

(III)

wobei $R_1$ und $R_2$ lineare oder verzweigte $C_1$-$C_4$-Alkylgruppen sind, oder miteinander verbunden werden können, um einen Ring von 3-10 Kohlenstoffatomen zu bilden; $R_4$ Wasserstoff oder eine $C_1$-$C_8$-Alkylgruppe ist;

$R_5$ eine lineare $C_1$-$C_{10}$-Alkylgruppe ist;
$R_6$ eine lineare $C_2$-$C_{20}$-Alkylgruppe ist;
$R_5$ und $R_6$ miteinander verbunden werden können, um einen Ring von 3-10 Kohlenstoffatomen zu bilden;
$R_7$ eine monoanionische Gruppe der Formel $-(CH_2)_mY$ ist, wobei m eine ganze Zahl von 0 bis 4 ist, und Y eine Carboxylat-, Sulfonat-, Sulfat-, Phosphonat- oder Phosphatgruppe ist;
mit der Maßgabe, dass die kombinierte Zahl von Kohlenstoffatomen $R_4$, $R_5$, $R_6$ und der zentralen Kohlenstoffatome 4 bis 22 beträgt.

**5.** Amphoteres Tensid gemäß Anspruch 4, wobei $R_4$ Wasserstoff ist.

**6.** Ein nichtionisches Tensid der Formel IV:

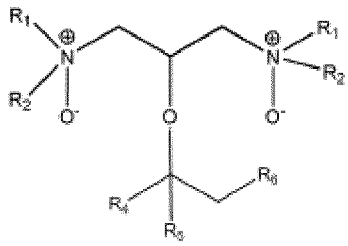

(IV)

wobei $R_1$ und $R_2$ lineare oder verzweigte $C_1$-$C_4$-Alkylgruppen sind, oder miteinander verbunden werden können, um einen Ring von 3-10 Kohlenstoffatomen zu bilden;

$R_4$ Wasserstoff oder eine $C_1$-$C_8$-Alkylgruppe ist;
$R_5$ eine lineare $C_1$-$C_{10}$-Alkylgruppe ist;
$R_6$ eine lineare $C_2$-$C_{20}$-Alkylgruppe ist;
$R_5$ und $R_6$ miteinander verbunden werden können, um einen Ring von 3-10 Kohlenstoffatomen zu bilden;
mit der Maßgabe, dass die kombinierte Zahl von Kohlenstoffatomen $R_4$, $R_5$, $R_6$ und der zentralen Kohlenstoffatome 4 bis 22 beträgt.

**7.** Nichtionisches Tensid gemäß Anspruch 6, wobei $R_4$ Wasserstoff ist.

**8.** Eine Zusammensetzung, die das kationische Tensid gemäß Anspruch 2 beinhaltet.

**9.** Zusammensetzung gemäß Anspruch 8 in Form eines Wäschereinigungsmittels, einer Farbe, einer Beschichtung, eines Emulsionspolymerisationsmittels, eines Haushalts- oder Industriereinigers, einer Landwirtschaftsformulierung, einer Latexformulierung, eines Umweltsanierungsmittels, einer Ölfeldchemikalie, einer Formulierung zur verbesserten Entölung, einer Formulierung zur Gasbehandlung, Textilverarbeitungs-, Appretur- oder Schlichtemittels, eines Pulpe- oder Papierverarbeitungsmittels, eines Duftstofflöslichkeitsmittels oder einer Duftstofflöslichkeitsformulierung, eines Fluids zur Metallverarbeitung oder eines Körperpflegeprodukts.

**10.** Zusammensetzung gemäß Anspruch 8 in Form eines Haarshampoos oder einer Haarspülung.

**11.** Ein Verfahren zur Behandlung von Haar, wobei das Verfahren das Anwenden einer Zusammensetzung gemäß Anspruch 10 beinhaltet.

**12.** Eine Zusammensetzung, die das amphotere Tensid gemäß Anspruch 4 beinhaltet.

**13.** Zusammensetzung gemäß Anspruch 12 in Form eines Wäschereinigungsmittels, einer Farbe, einer Beschichtung, eines Emulsionspolymerisationsmittels, eines Haushalts- oder Industriereinigers, einer Landwirtschaftsformulierung, einer Latexformulierung, eines Umweltsanierungsmittels, einer Ölfeldchemikalie, einer Formulierung zur verbesserten Entölung, einer Formulierung zur Gasbehandlung, Textilverarbeitungs-, Appretur- oder Schlichtemittels, eines Pulpe- oder Papierverarbeitungsmittels, eines Duftstofflöslichkeitsmittels oder einer Duftstofflöslichkeitsformulierung, eines Fluids zur Metallverarbeitung oder eines Körperpflegeprodukts.

**14.** Eine Zusammensetzung, die das nichtionische Tensid gemäß Anspruch 6 beinhaltet.

**15.** Zusammensetzung gemäß Anspruch 14 in Form eines Wäschereinigungsmittels, einer Farbe, einer Beschichtung, eines Emulsionspolymerisationsmittels, eines Haushalts- oder Industriereinigers, einer Landwirtschaftsformulierung, einer Latexformulierung, eines Umweltsanierungsmittels, einer Ölfeldchemikalie, einer Formulierung zur verbesserten Entölung, einer Formulierung zur Gasbehandlung, Textilverarbeitungs-, Appretur- oder Schlichtemittels, eines Pulpe- oder Papierverarbeitungsmittels, eines Duftstofflöslichkeitsmittels oder einer Duftstofflöslichkeitsformulierung, eines Fluids zur Metallverarbeitung oder eines Körperpflegeprodukts.

## Revendications

**1.** Une diamine de formule I.

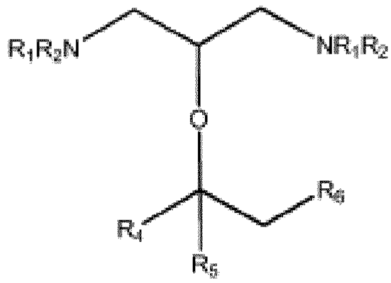

(I)

dans laquelle $R_1$ et $R_2$ sont des groupes méthyle, éthyle ou butyle, ou peuvent être reliés l'un à l'autre pour former un cycle de 3 à 9 atomes de carbone ;

$R_4$ est un hydrogène ou un groupe alkyle en $C_1$ à $C_8$ ;
$R_5$ est un groupe alkyle linéaire en $C_1$ à $C_{10}$ ;
$R_6$ est un groupe alkyle en $C_2$ à $C_{20}$ linéaire ;
$R_5$ et $R_6$ peuvent être reliés l'un à l'autre pour former un cycle de 3 à 10 atomes de carbone ;
à la condition que le compte des atomes de carbone combinés de $R_4$, $R_5$, $R_6$ et de l'atome de carbone central soit de 4 à 22.

**2.** Un tensioactif cationique de formule II :

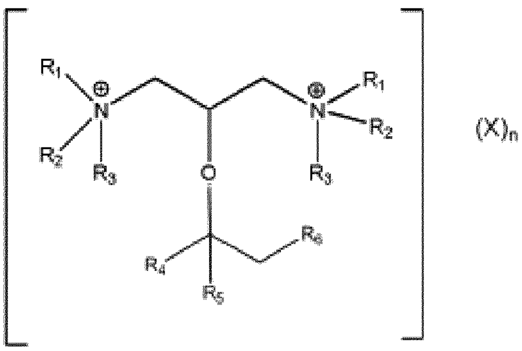

(II)

dans laquelle $R_1$ et $R_2$ sont des groupes alkyle linéaires ou ramifiés en $C_1$ à $C_4$, ou peuvent être reliés l'un à l'autre pour former un cycle de 3 à 9 atomes de carbone ;

$R_3$ est un groupe arylalkyle ou alkyle linéaire ou ramifié en $C_1$ à $C_{20}$ ;
$R_4$ est un hydrogène ou un groupe alkyle en $C_1$ à $C_8$ ;
$R_5$ est un groupe alkyle linéaire en $C_1$ à $C_{10}$ ;
$R_6$ est un groupe alkyle en $C_2$ à $C_{20}$ linéaire ;
$R_5$ et $R_6$ peuvent être reliés l'un à l'autre pour former un cycle de 3 à 10 carbones ;
à la condition que le compte des atomes de carbone combinés de $R_4$, $R_5$, $R_6$ et de l'atome de carbone central soit de 4 à 22 ;
X est un mono- ou di-anion d'au moins l'un parmi l'halogénure, le bicarbonate, le carbonate, le sulfate, le sulfate de méthyle et le bisulfate ; et
n est un nombre entier tel que la charge anionique totale fournie par $X_n$ soit de 2.

**3.** Le tensioactif cationique de la revendication 2 dans lequel $R_4$ est un hydrogène.

**4.** Un tensioactif amphotère de formule III :

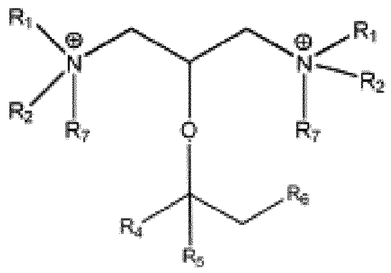

(III)

dans laquelle $R_1$ et $R_2$ sont des groupes alkyle linéaires ou ramifiés en $C_1$ à $C_4$, ou peuvent être reliés pour former un cycle de 3 à 10 atomes de carbone ;

$R_4$ est un hydrogène ou un groupe alkyle en $C_1$ à $C_8$ ;
$R_5$ est un groupe alkyle linéaire en $C_1$ à $C_{10}$ ;
$R_6$ est un groupe alkyle en $C_2$ à $C_{20}$ linéaire ;
$R_5$ et $R_6$ peuvent être reliés l'un à l'autre pour former un cycle de 3 à 10 atomes de carbone ;
$R_7$ est un groupe mono-anionique de la formule $-(CH2)_mY$, où m est un nombre entier de 0 à 4, et Y est un groupe carboxylate, sulfonate, sulfate, phosphonate, ou phosphate ;
à la condition que le compte des atomes de carbone combinés de $R_4$, $R_5$, $R_6$ et de l'atome de carbone central soit de 4 à 22.

**5.** Le tensioactif amphotère de la revendication 4 dans lequel $R_4$ est un hydrogène.

**6.** Un tensioactif non ionique de formule IV :

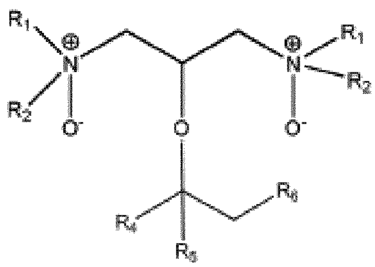

(IV)

dans laquelle $R_1$ et $R_2$ sont des groupes alkyle linéaires ou ramifiés en $C_1$ à $C_4$, ou peuvent être reliés pour former un cycle de 3 à 10 atomes de carbone ;

$R_4$ est un hydrogène ou un groupe alkyle en $C_1$ à $C_8$ ;
$R_5$ est un groupe alkyle linéaire en $C_1$ à $C_{10}$ ;
$R_6$ est un groupe alkyle en $C_2$ à $C_{20}$ linéaire ;
$R_5$ et $R_6$ peuvent être reliés l'un à l'autre pour former un cycle de 3 à 10 atomes de carbone ;
à la condition que le compte des atomes de carbone combinés de $R_4$, $R_5$, $R_6$ et de l'atome de carbone central soit de 4 à 22.

**7.** Le tensioactif non ionique de la revendication 6 dans lequel $R_4$ est un hydrogène.

**8.** Une composition comprenant le tensioactif cationique de la revendication 2.

**9.** La composition de la revendication 8 sous la forme d'un détergent à lessive, d'une peinture, d'un enduit, d'un agent de polymérisation en émulsion, d'un nettoyant ménager ou industriel, d'une formulation agricole, d'une formulation de latex, d'un agent d'assainissement de l'environnement, d'un produit chimique de champ pétrolifère, d'une formulation de récupération assistée du pétrole, d'une formulation de traitement du gaz, d'un agent de transformation, de finition ou d'ensimage des textiles, d'un agent de fabrication de pâte ou de papier, d'un agent ou d'une formulation de solubilisation de fragrance, d'un fluide d'usinage de métaux ou d'un produit de soins personnels.

**10.** La composition de la revendication 8 sous la forme d'un shampooing ou d'un après-shampooing.

**11.** Une méthode de traitement des cheveux, la méthode comprenant l'application d'une composition de la revendication 10.

**12.** Une composition comprenant le tensioactif amphotère de la revendication 4.

**13.** La composition de la revendication 12 sous la forme d'un détergent à lessive, d'une peinture, d'un enduit, d'un agent de polymérisation en émulsion, d'un nettoyant ménager ou industriel, d'une formulation agricole, d'une formulation de latex, d'un agent d'assainissement de l'environnement, d'un produit chimique de champ pétrolifère, d'une formulation de récupération assistée du pétrole, d'une formulation de traitement du gaz, d'un agent de transformation, de finition ou d'ensimage des textiles, d'un agent de fabrication de pâte ou de papier, d'un agent ou d'une formulation de solubilisation de fragrance, d'un fluide d'usinage de métaux ou d'un produit de soins personnels.

**14.** Une composition comprenant le tensioactif non ionique de la revendication 6.

**15.** La composition de la revendication 14 sous la forme d'un détergent à lessive, d'une peinture, d'un enduit, d'un agent de polymérisation en émulsion, d'un nettoyant ménager ou industriel, d'une formulation agricole, d'une formulation de latex, d'un agent d'assainissement de l'environnement, d'un produit chimique de champ pétrolifère, d'une formulation de récupération assistée du pétrole, d'une formulation de traitement du gaz, d'un agent de transformation, de finition ou d'ensimage des textiles, d'un agent de fabrication de pâte ou de papier, d'un agent ou d'une formulation de solubilisation de fragrance, d'un fluide d'usinage de métaux ou d'un produit de soins personnels.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3959461 A
- US 5705681 A
- US 20090281359 A